(19) Europäisches Patentamt
European Patent Office
Office européen des brevets

(11) **EP 3 488 779 B1**

(12) **EUROPEAN PATENT SPECIFICATION**

(45) Date of publication and mention
of the grant of the patent:
**23.09.2020 Bulletin 2020/39**

(51) Int Cl.:
*A61B 5/06* *(2006.01)*          *A61B 5/00* *(2006.01)*
*A61B 34/20* *(2016.01)*

(21) Application number: **18208600.9**

(22) Date of filing: **27.11.2018**

(54) **LOW PROFILE DUAL PAD MAGNETIC FIELD LOCATION SYSTEM WITH SELF TRACKING**

NIEDERPROFIL-DOPPELPOLSTERMAGNETFELDLOKALISIERUNGSSYSTEM MIT SELBSTVERFOLGUNG

SYSTÈME DE LOCALISATION DE CHAMP MAGNÉTIQUE À DOUBLE TAMPON, À PROFIL BAS ET À SUIVI AUTOMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2017 US 201762591238 P**
**12.09.2018 US 201816129263**

(43) Date of publication of application:
**29.05.2019 Bulletin 2019/22**

(60) Divisional application:
**20175066.8**

(73) Proprietor: **Biosense Webster (Israel) Ltd.**
**Yokneam 2066717 (IL)**

(72) Inventors:
• **DEMRI, Tamir**
**2010300 Gilon (IL)**
• **ELDAR, Ranit**
**4645511 Herzliya (IL)**
• **BLATT, Ofir**
**2018100 Oranit (IL)**
• **MONTAG, Avram Dan**
**2066717 Yokneam (IL)**

(74) Representative: **Small, Gary James**
**Carpmaels & Ransford LLP**
**One Southampton Row**
**London WC1B 5HA (GB)**

(56) References cited:
**EP-A1- 0 883 374       EP-A1- 1 570 781**
**US-A1- 2006 025 668       US-A1- 2017 196 477**

## Description

### SUMMARY

A magnetic field location system ; is provided for use in tracking a distal end of a catheter or the like in a surgical or exploratory procedure within a living body disposed on a support structure, such as a patient bed, table or gurney. Dual low-profile elongated field generation pads are provided that are configured for selective placement along respective sides of the living body disposed on the support structure. Each field generation pad includes a plurality of magnetic coils operable to generate magnetic fields.

A securing structure is provided to releasably secure the dual field generation pads to the support structure when selectively placed on the support structure. The dual field generation pads and associated securing structure are preferably configured such that the dual field generation pads are able to be selectively placed on and secured to the support structure in less than 60 seconds and are also able to be removed from the support structure in less than 60 seconds. Preferably, each of the first and second elongated field generation pads are configured with a low-profile height not exceeding 20 mm and a weight not exceeding 3 Kg.

A processor is coupled to the magnetic coils of the dual field generation pads. The processor is configured to execute a process for correlation of magnetic fields generated by the field generation pads such that a reference magnetic field is defined that extends within a desired portion of the living body disposed on the support structure. This enables precise location and tracking of a distal end of a catheter via a magnetic sensor of the catheter within the reference magnetic field.

The dual field generation pads are preferably relatively symmetric and define relatively symmetric magnetic coil arrays when placed by the sides of the living body. In one embodiment, the magnetic fields generated by the dual field generation pads are generated by two magnetic coils disposed in each field generation pad at a defined distance from each other. In such an embodiment, each magnetic coil can be configured as a tri-axial coil unit, such as tri-coil bobbins that have a combined weight of approximately 200 grams. In another embodiment, the magnetic fields generated by the dual field generation pads are generated by six linearly aligned and equally spaced magnetic coils disposed in each field generation pad, where each magnetic coil is configured as a single coil unit.

To secure each field generation pad to the support structure, two securing mechanisms can be attached to each field generation pad at predefined locations spaced apart from each other. In such case, each securing mechanism can have an arm having an end attached to the respective field generation pad and a support structure engaging portion. The support structure engaging portion of the arm can be configured such that the support structure engaging portion engages a side of the support structure when the respective field generation pad is selectively placed on the support structure, thereby defining the relative placement of the respective field generation pad on the support structure.

The pad attaching end of the arm of each such securing mechanism may have an adjustable coupling to enable the positioning of the respective support structure engaging portion at an extended or a retracted position relative to the respective field generation pad. As such, the dual field generation pads are placed furthest from each other and closest to the sides of the support structure when all of the support structure engaging portions are in the retracted position and are placed closest to each other and furthest from the sides of the support structure when all of the support structure engaging portions are in the extended position.

In one embodiment, each securing mechanism has a clamp configured to be operated to a clamping position from an open position. The clamps are preferably operated when the respective support structure engaging portions are engaged with respective sides of the support structure so that field generation pads are secured in their selectively placed positions on the support structure. In another embodiment, each securing mechanism may have a coupling component configured for connection beneath the support structure. In such case, the securing structure can include a first joining member configured to attachment to the coupling components of the first securing mechanisms and a second joining member configured for attachment to the coupling components of the second securing mechanism to secure the selective placement of the field generation pads on the support structure when the joining member are attached.

These and other objects, features and advantages of the present invention will be apparent from the following detailed description of illustrative embodiments thereof, which is to be read in connection with the accompanying drawings.

BRIEF DESCRIPTION OF THE DRAWINGS

The present invention is illustrated by way of example, and not by way of limitation, in the figures of the accompanying drawings.

Figure 1 is example schematic, pictorial illustration of a magnetic location system for use in tracking a distal end of a catheter of the like within a living subject during a medical procedure.

Figure 2A is a perspective view of an embodiment the magnetic location system comprising dual low-profile elongated field generation pads secured to a patient table.

Figure 2B is a top view of the dual low-profile elongated field generation pads shown in Fig. 2A.

Figure 2C is a bottom view of the embodiment of the magnetic location system shown in Fig. 2A.

Figure 3 is a perspective view of the of magnetic location system shown in Fig. 2A with an installable arm rest.

Figure 4A is a top view of another embodiment of one of the dual low-profile elongated field generation pads shown in Fig. 2A illustrating an array of rod-connected field generating coil units in phantom.

Figure 4B is a cross section of the low-profile elongated field generation pad along line 4B of Fig. 4A.

Figure 5A is a perspective view of the securing mechanism for the dual low-profile elongated field generation pads of Fig. 2A in an "open" position.

Figure 5B is a side view of the securing mechanism for the dual low-profile elongated field generation pads of Fig. 2A in the "open" position.

Figure 6A is a perspective view of the securing mechanism for the dual low-profile elongated field generation pads of Fig. 2A in a "clamping" position.

Figure 6B is a side view of the securing mechanism for the dual low-profile elongated field generation pads of Fig. 2A in the "clamping" position.

Figure 7 is a bottom view of a portion of one of the dual low-profile elongated field generation pads of Fig. 2A.

Figure 8A is an end view of another embodiment of a securing mechanism for the dual low-profile elongated field generation pads of Fig. 2A.

Figure 8B is a bottom perspective view of the securing mechanism for the dual low-profile elongated field generation pads of Fig. 8A.

Figure 9 is a bottom view of another alternative of a securing mechanism for the dual low-profile elongated field generation pads of Fig. 2A.

## DETAILED DESCRIPTION

Various methods and systems are known in the art for tracking the coordinates of objects involved in medical procedures. For example, magnetic navigation systems may be used for navigating tools, such as medical implements, catheters, wireless devices, etc. One such magnetic navigation system can be found in the CARTO® system, produced by Biosense Webster, Inc. (Diamond Bar, California).

In magnetic navigation systems, magnetic fields are, for example, generated by a location pad that includes a plurality of magnetic field generators. In a magnetic navigation system, magnetic position sensing may be used to determine position coordinates of the distal end of a tool inside a patient's organ. For this purpose, a driver circuit in a console or a location pad drives the plurality of field generators to generate magnetic fields that extend within the body of a patient.

Typically, the field generators comprise a plurality of coils, which are, for example in the CARTO™ system, incorporated into a unitary location pad that is placed below the patient's torso at known position external to the patient, such as below the patient table. The unitary pad system was a significant improvement over the direct application of individual magnetic field generation coils to a patient's torso such as disclosed in U.S. Pat. No. 6,618,612, where coil placement was more cumbersome, could interfere with a performance of a desired medical procedure, and could require disinfectant cleaning for re-use.

The coils generate magnetic fields in a predefined working volume that contains the patient's organ to be explored. One or more magnetic field sensors located on a medical tool detect the magnetic fields and generate electrical signals in response to these magnetic fields. A signal processor processes these signals in order to determine the position coordinates of the medical tool, typically including both location and orientation coordinates. Each coil transmits at a different frequency so that the processor can separate the magnetic fields from each source. This method of position sensing is implemented in the above-mentioned CARTO™ system and is described in detail in U.S. Pat. Nos. 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

The CARTO® system has conventionally employed a unitary location pad for generating the magnetic field used for magnetic location sensing. The unitary pad contains multiple magnetic coils that create a magnetic field and is generally placed beneath the patient. The unitary pad generates an ultralow magnetic reference field from which the location of the tool (having a magnetic field sensor) can be determined. This provides the physician with visibility and orientation of the patient's internal anatomy. The construction of suitable magnetic coils, i. e. reference field transducers, is disclosed, for example, in U.S. Pat. No. 6,618,612.

Further magnetic field location systems for use in tracking a medical instrument are disclosed in EP 0883374 B1, US 2017/0196477 A1, US 2006/0025668 A1, and EP 1570781 A1.

The inventive location system described herein features magnetic field generator coil units 201 disposed in dual low-profile elongated field generation pads 202 that are easier to install on the top of a patient table, such as a bed or gurney 28, on either side of the patient's torso, than currently existing unitary pad or individual field generator systems. The system also includes a self-tracking algorithm to define the magnetic reference field used by a catheter sensor when implementing location sensing and to make installation easier. An example, self-tracking algorithm for the pads is discussed below.

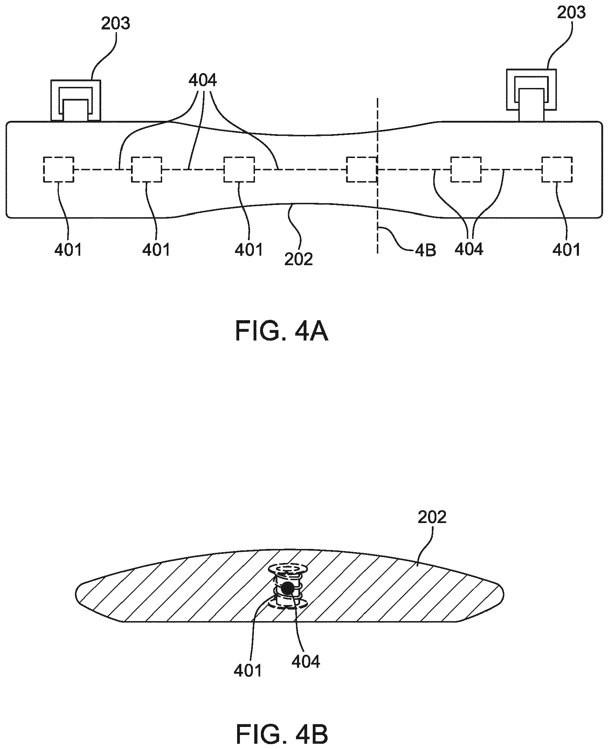

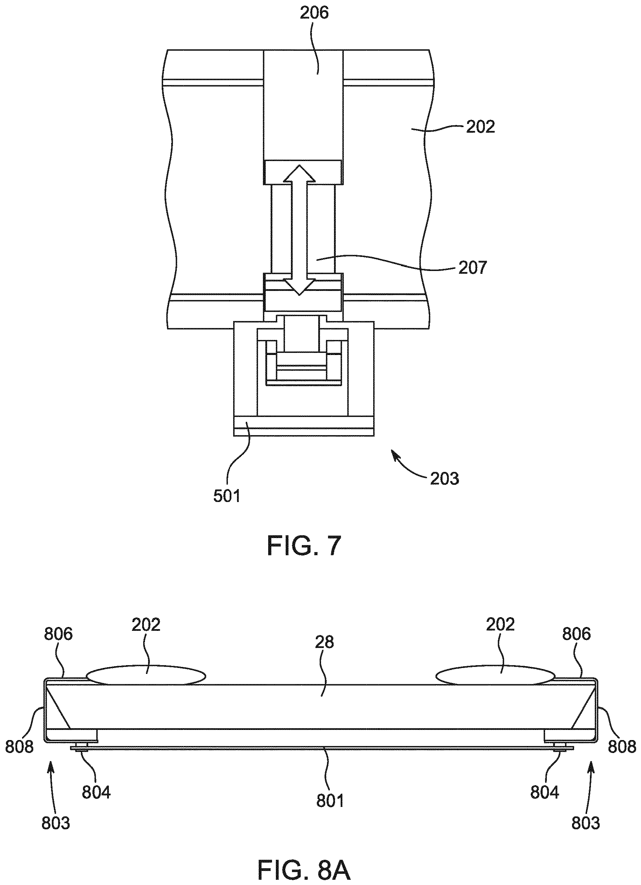

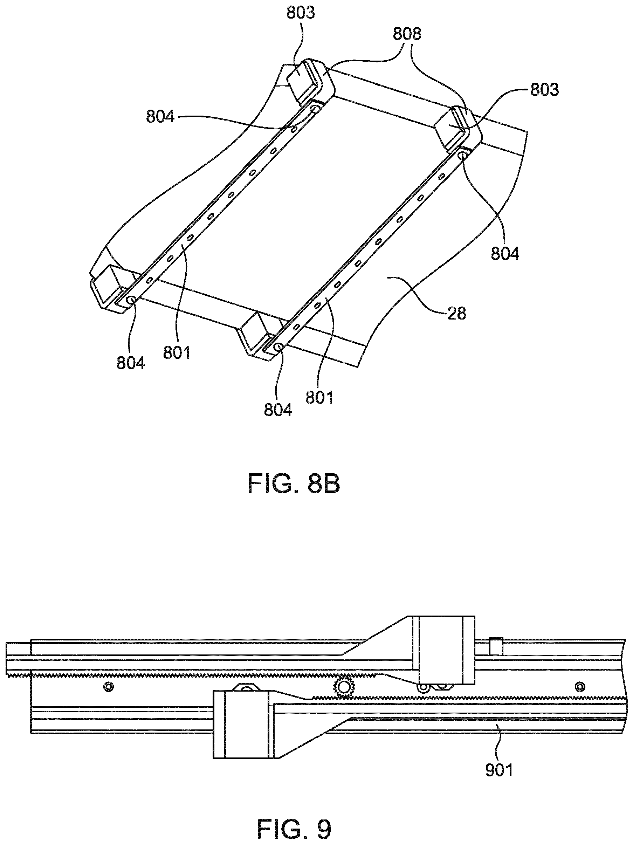

A clamping mechanism, such as clamps 203 of Figs. 4A-7, can be used to secure the field generation pads 202 in place for use. As an alternative, a flexible band, such as elastic member 801 of Fig. 8A-B, can be used for securing the field generation sensor pads 202 on a patient's bed. In lieu of the elastic members 801, a rack and pinion mechanism 901, such as illustrated in Fig. 9, may be employed.

In particular, a first embodiment employs dual low-profile elongated field generation pads 202 rather than the triangular location pad currently used in the CARTO™ system or individual coil units. The dual low-profile elongated field generation pads 202 are preferably situated on an axis, such as the longitudinal axis, of the patient table 28 on either side of a patient and secured in place by a securing structure attach them to the patient table 28. The securing structure is designed to prevent the dual low-profile elongated field generation pads 202 from moving during the medical or exploratory procedure.

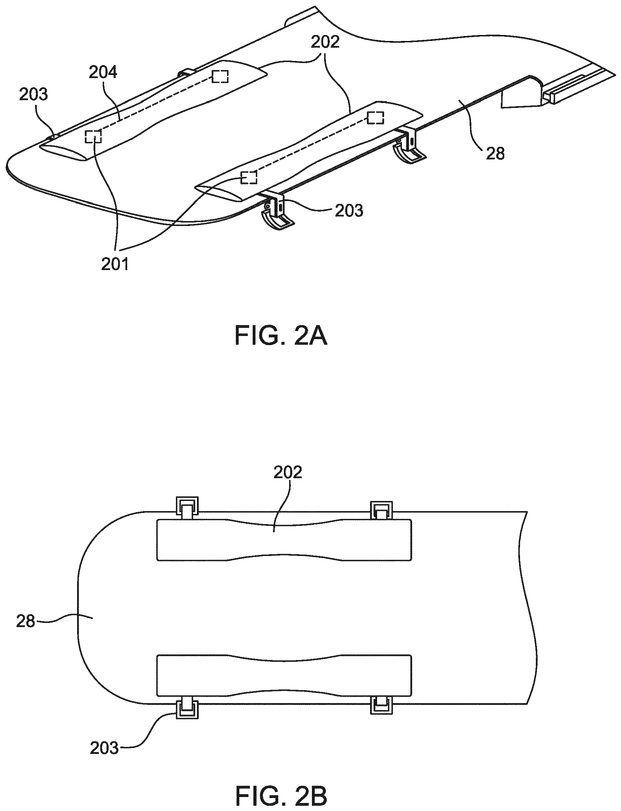

Clamping mechanisms 203 are provided as the securing structure for the embodiment illustrated in Figs 2A-7. The clamping mechanisms 203 allow a physician and/or location pad installer to add the dual low-profile elongated field generation pads 202 before the procedure starts and/or during the procedure. Thus, the patient barely needs to move to add the dual low-profile elongated field generation pads 202 whereas the known unitary location pad and individual coil configurations require considerable effort to allow the location pad or individual coil units to be appropriately positioned. Situating the dual low-profile elongated field generation pads 202 on the table 28 permits the field generating coils 201 to be close to the patient to generate magnetic field gradients for assuring location accuracy and to be distanced from items such as fluoroscopy system collimators which are undesired metal sources that interfere with the magnetic location system.

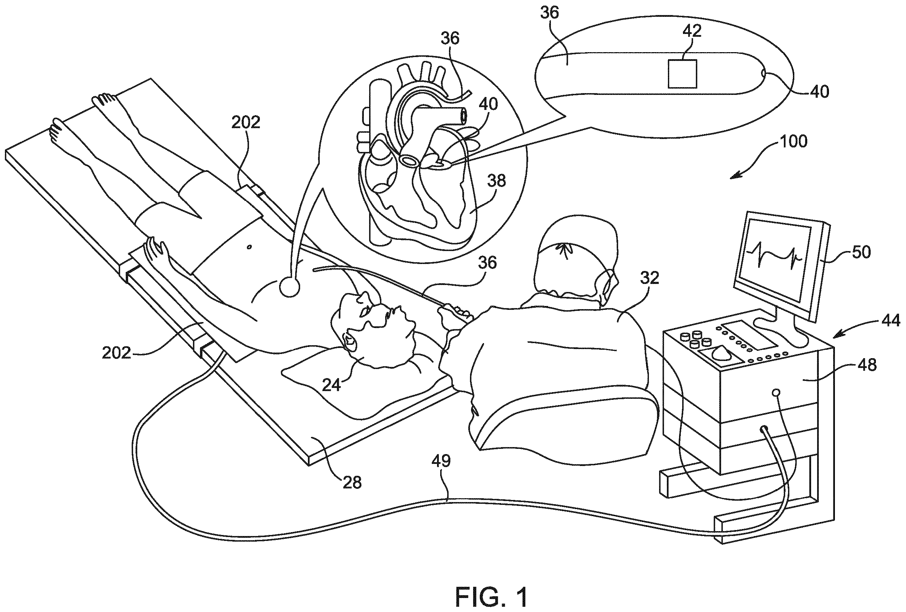

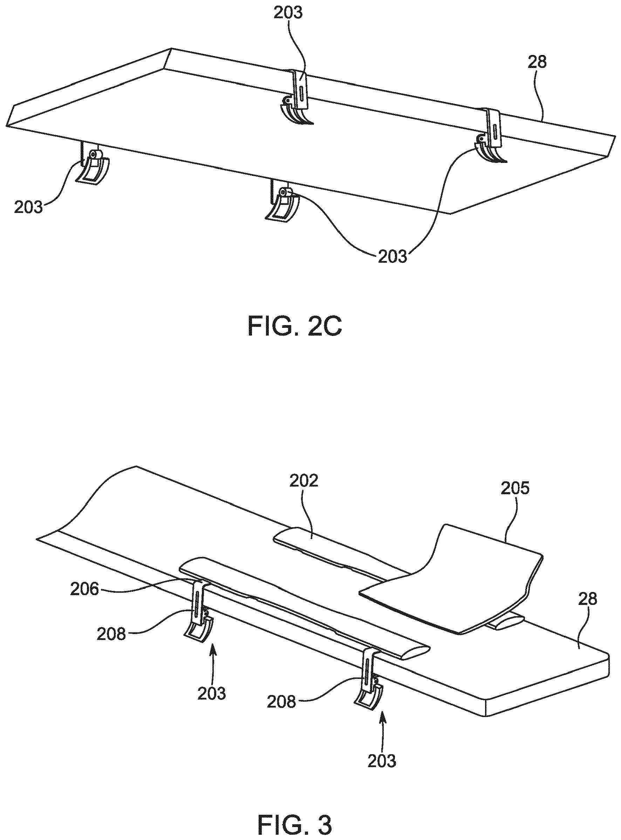

Figure 1 is a schematic, pictorial illustration of a magnetic position tracking system 100 used in cardiac catheterization applications, in accordance with an embodiment of the present invention. System 100 comprises dual low-profile elongated field generation pads 202, which comprises one or more field generators, such as field generating coils. Embodiments of the dual low-profile elongated field generation pads 202 are described in more detail with respect to Figures 2-9.

A patient 24 lies on a catheterization or patient table 28. A physician 32 inserts a catheter 36 into a chamber of a heart 38 (shown as balloon insert) of the patient 24. System 100 determines and displays the position and orientation coordinates a distal end 40 of catheter 36 inside the heart 38.

In the exemplary configuration of Figure 1, the dual low-profile elongated field generation pads 202 comprise embedded field generating coil units 201, 401 see Figures 2A, 4A and 4B. The dual low-profile elongated field generation pads 202 are placed on top of the table 28 along or under the patient's torso 24, such that the coils 201 become located in fixed positions external to the patient 24 and are operable to generate magnetic fields in a defined working volume around the heart. A field definition algorithm is implemented once the pads are positioned which defines the magnetic field for sensor use.

A position sensor 42 fitted in the distal end 40 of the catheter 36 senses the magnetic fields in its vicinity. The position sensor 42 produces and transmits, in response to the sensed fields, position signals to a console 44. The console 44 comprises a tracking processor 48 that calculates the location and orientation of catheter 36 with respect to the coils 201, based on the position signals sent by position sensor 42. Typically, the console 44 also drives the coils 201 through a connector cable 49 to generate the appropriate magnetic fields and to implement the field definition algorithm. The location and orientation coordinates of catheter 36 are displayed to the physician using a display 50.

Although Figure 1 shows an example system 100 for cardiac catheterization or other procedures, the dual low-profile elongated field generation pads 202 can be used in other position tracking applications, such as for tracking orthopedic implants and other medical tools.

The dual low-profile elongated field generation pads 202 may also be compatible with fluoroscopy systems used by the magnetic tracking system. Accordingly, in one embodiment, the dual low-profile elongated field generation pads 202 may be radiolucent when centered in a horizontal plane below a Fluoro iso-center for a Fluoro C-arm orientation of LA060 - RAO60 (@CRA/CAU 0). Also, the dual low-profile elongated field generation pads 202 may be designed to minimize interference with the Fluoro C-arm movement (considering LAO-RAO rotation). The dual low-profile elongated field generation pads 202 may be robust and flexible to withstand mechanical shocks, such as a drop of up to one meter onto a hard surface (e.g., floor), without damage.

The functional interface of the dual low-profile elongated field generation pads 202 with the tracking processor 48 of the system 100 may be implemented through a wireless connection. In that case, the location pad will be connected only to a power supply through a power cable in lieu of the connector cable 49. The wireless connection can be based on standard wireless protocols (such as WLAN IEEE802.11) + implementation for synchronization.

Figure 2A illustrates example dual low-profile elongated field generation pads 202 positioned along the longitudinal axis of the patient table 28. In this example, the dual low-profile elongated field generation pads 202 have four tri-coils magnetic field generation units 201, two for each pad 202. For each pad, the field generation units 201 are disposed at opposite ends the pad 202 at a fixed distance such that the pad's two field generation coil units 201 are preferably spaced no greater than 500 mm from the center of each other. The distance is selected to assure sufficient magnetic field generation using relatively low profile field generation coil units 201. To maintain the fixed spacing of the coil units within each pad, a connecting rod 204 can be provided having ends secured to each field generation coil unit 201 within the pad 202.

In the Figure 2A example, the field generation coil units 201 are preferably tri-coil bobbins that weigh about 200 grams together so that each pad weighs only 1-2 Kg and is 540 mm in length, 140 mm in width and 8.5 mm in height. As such, the dual low-profile elongated field generation pads 202 are easy to install, have a low profile and low weight compared to existing systems. To permit relatively easy handling and placement of the pads 202, they generally do not exceed 3 Kg in weight and do not have a height exceeding 20 mm.

The dual low-profile elongated field generation pads 202 are installed to produce a magnetic field that defines a desired magnetic field zone in the system 100. In above embodiment, the distance between the dual low-profile elongated field generation pads 202 when installed preferably does not exceed 50 cm to maintain sufficient magnetic field gradients within the working volume.

In connection with installing the pads 202, the tracking processor 48 can be programmed to detect if the pads 202 are positioned within a predetermined maximum distance, such as 50 cm, of each other. The processor can generate a signal that is relayed to the console to display an indication on the display 50 as to whether the pads 202 are positioned close enough to each other to properly generate the magnetic sensing field.

Preferably the pads are symmetric to each other with each having a same number of field generation coil units in each spatial direction, typically multiples of three, are used. The dual low-profile elongated field generation pads 202 may support Improved "Single Axis Sensor" (SAS) accuracy. In contrast to the Figure 2A embodiment, where tri-axial coils 201 are disposed at each end of each of the pads 202, Figs. 4A and 4B illustrates in another embodiment, wherein an array of six single axis coils 401 are equally distributed along the length of each of the dual low-profile elongated field generation pads 202. Connecting rod members 404 are provided to maintain an equal fixed spacing between the single axis field generation coils 401 within each pad 202.

The dual low-profile elongated field generation pads 202 include a securing mechanism, such as clamps 203, to secure the pads 202 to the patient table 28 to prevent unintentional movement of the pads 202 such as a result of lateral force lower than about 100 Newtons. In one embodiment, the dual low-profile elongated field generation pads 202 have an ergonomic design that is in line with the design elements of the entire system to emphasize the fact that dual low-profile elongated field generation pads 202 are parts of one system 100.

The securing structure, such as clamp mechanisms 203, is preferably designed to permit installation of the dual low-profile elongated field generation pads 202 within one minute and uninstallation in even less time. In busy surgical practices, patient surgical operations, mappings and/or other procedures are often performed on a relatively tight time schedule. The present invention greatly facilitates the efficient conduct of such successive procedures by permitting the very rapid uninstallation of the field generation pads from the table of a patient who had undergone a procedure and installation of the pads on the table of another patient scheduled for a next procedure.

The location pad 20 may comprise a holding structure that avoids interference with a patient table arm rest 205 on the patient table 28. As shown in Figure 3, in one embodiment, the field generation pad 202 may be integrated with the arm rest 205 as a single device.

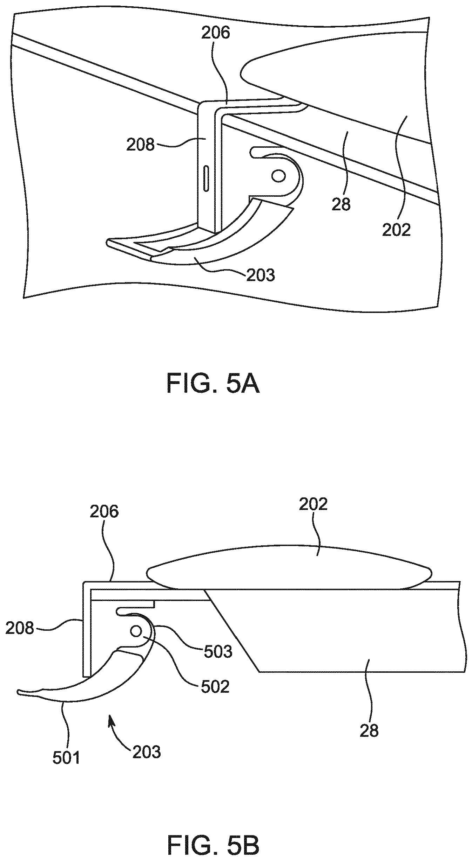

Figures 2A-7 illustrate a simple clamp type mechanism 203 for securing the low-profile elongated field generation pads 202 to the patient table 28. In this example, each pad is provided with two clamp mechanisms 203. As best seen in Figures 5A-6B, the securing clamp mechanisms 203 each include a pad attaching arm 206 connected to a respective field generation pad 202. The arms 206 each have a table engaging portion 208 which is configured to abut against a side of the patient table 28 when the pad 202 is installed so that the engagement of the table engaging portions 208 define the location of the pads 202 relative to the table 28, as best seen in Figures 2A and 2B.

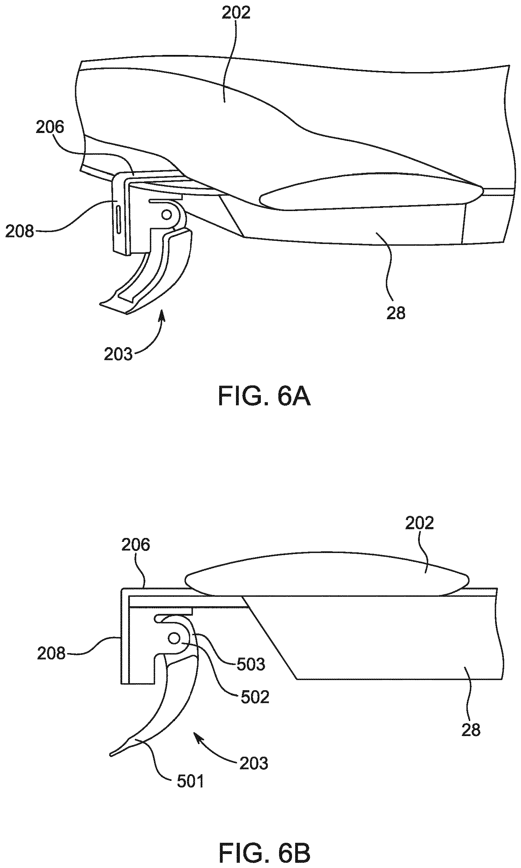

Figures 5A and 5B illustrate views of one of the clamping mechanisms 203 in an open state that enables the field generation pad 202 to be positioned on the table 28 so that the table engaging portion 208 of each clamp mechanism 203 abuts a respective side of the table 28. Figures 6A and 6B illustrate views of the clamping mechanism 203 in a clamping state with the field generation pad 202 appropriately positioned on the table 28.

The example clamping mechanisms 203 are of a lever type design, each having a handle portion 501 that can be easily gripped to toggle between the open and clamping positions by an individual installing the field generation pad 202. The lever handle 501 of the clamping mechanism 203 pivots about a pivot shaft 502 to displace a clamping end portion 503 between clamping and open positions. As shown in Figures 6A and 6B, the handle 501 has been pushed downward which pivots the clamping end portion 503 upwards against the bottom of the patient table. In this clamping position, enough force is exerted on the patient table so that the attached field generation pad 202 is not easily moveable.

Figure 7 shows a bottom view of a portion of one of the field generation pads 202 illustrating the optional adjustability of the attachment of the arm 206 of the clamping mechanism 203 to the pad 202. The arm 206 is equipped with an adjustable coupling 207 which allows adjustment of the distance the table engaging portion 208 from the field generation pad 202. The adjustable coupling 207 can be configured, for example, as a slot/tab mechanism, a slide type mechanism, or projecting pins that are engageable in a variety of locations with respect to a series of defined holes.

The adjustable coupling 207 enables the table engaging portion 208 of the arm 206 of the securing mechanism 203 to be positioned at an extended position or a retracted position relative to the pad 202 and may be configured to permit the attachment of the arm 206 to the table engaging portion 208 at one or more positions therebetween. When installed on the table 28, the dual field generation pads 202 will be furthest from the respective sides of the table 28 and closest to each other when the table engaging portions 208 of the securing mechanisms 203 are in the extended position. When the table engaging portions 208 of the securing mechanisms 203 are in the retracted position, the dual field generation pads 202 will be closest to the respective sides of the table 28 and furthest from each other when installed. The adjustability of the coupling of the securing mechanisms 203 to the pads 202 enables use of the pads with both wide and narrow tables.

Figures 8A and 8B illustrate an alternative embodiment of a table securing mechanism 803 for the field generation pads 202. The securing mechanisms 803 each include a pad attaching arm 806 along with a table engaging portion 808 similar to the arm 206 and table engaging portion 208 of the clamping mechanism 203 described above. In addition, each securing mechanism 803 includes a boss 804 mounted on a portion of the arm 806 that extends to the underside of the table 28. The boss 804 is configured to receive an end of an elastic member or the like 801. The elastic member 801 serves to secure the field generation pads 202 in position on the patient table 28. The arm 806 may include an adjustable coupling, such as arm coupling 207 of clamping mechanism 203, to accommodate different table widths when the securing mechanisms 803 are used. Elastic member 801 may be made of rubber or other elastic material of appropriate length to exert a sufficient securing tension to prevent inadvertent movement of the pads 202 when installed on the patient table 28.

As shown in the example illustrated in Fig. 8B, when the dual field generation pads 202 are installed, each pad 202 is secured to the table by two securing mechanisms 803 which each oppose a respective securing mechanism 803 of the other pad 202. Two elastic members 801 are used, each one securing a respective opposing pair of securing mechanisms 803.

In lieu of the elastic member 801, a rack and pinion mechanism 901, such as shown in Fig. 9, or other connecting device may be used to connect the securing mechanisms 803 of opposing dual field generation pads 202. In an embodiment when the joining member is not elastic, it may also have visible distance markings (i.e. mm or cm) or known lengths in order to assure that the dual pads 202 are within a desired maximum distance from each other.

As referenced above, a self-tracking process is performed to determine where the dual field generation pads 202 are co-located. This self-tracking enables the use of the two pads 202 whose physical proximity may change during the course of a medical procedure and/or during the set-up for a medical procedure. This tracking uses the position of the components relative to one another. Each component has at least one transmitter and sensor to communicate with the other components. Each pad 202 includes sensors that read the magnetic field induced by the transmitting coils of the other pad. Such sensors may be incorporated into the field generation units 201, for example, see U.S. Pat. No. 6,618,612. These readings enable one to learn the relative position of the two pads.

The self-tracking algorithm provides the locations and orientations of the transmitting coils in a defined coordinate system. The transmitting coils must have been previously calibrated, that is their magnetic moments (up to any desired order of spherical harmonics) must be known. There are many ways to define the coordinate system. For concreteness, a coordinate system whose origin is the centroid of the location of all the transmitting coils can be chosen. The x-y plane is defined by a plane containing the centers of any two coils of one of the pads 202 and the center of a coil on the other pad 202. In an example of working to order 1 (dipole) in a spherical harmonics expansion, the concept can be readily extended to any order, a rotation matrix can be defined as

$$Rot[\varphi,\theta,\psi]$$
$$= \begin{bmatrix} \cos(\theta)\cos(\phi) & \cos(\theta)\sin(\phi) & -1.\sin(\theta) \\ \cos(\phi)\sin(\theta)\sin(\psi) - 1.\cos(\psi)\sin(\phi) & \cos(\phi)\cos(\psi) + \sin(\theta)\sin(\phi)\sin(\psi) & \cos(\theta)\sin(\psi) \\ \cos(\phi)\cos(\psi)\sin(\theta) + \sin(\phi)\sin(\psi) & \cos(\psi)\sin(\theta)\sin(\phi) - 1.\cos(\phi)\sin(\psi) & \cos(\theta)\cos(\psi) \end{bmatrix}$$

and a dipole polynomial matrix as

$$bDipol[x,y,z] = \begin{bmatrix} \dfrac{-2x^2+y^2+z^2}{(x^2+y^2+z^2)^{5/2}} & -\dfrac{3xy}{(x^2+y^2+z^2)^{5/2}} & -\dfrac{3xz}{(x^2+y^2+z^2)^{5/2}} \\[3mm] -\dfrac{3xy}{(x^2+y^2+z^2)^{5/2}} & \dfrac{x^2-2y^2+z^2}{(x^2+y^2+z^2)^{5/2}} & -\dfrac{3yz}{(x^2+y^2+z^2)^{5/2}} \\[3mm] -\dfrac{3xz}{(x^2+y^2+z^2)^{5/2}} & -\dfrac{3yz}{(x^2+y^2+z^2)^{5/2}} & \dfrac{x^2+y^2-2z^2}{(x^2+y^2+z^2)^{5/2}} \end{bmatrix}$$

Let $(x_i, y_i, z_i)$ be the location of transmitter i and $(x_j, y_j, z_j)$ be the location of transmitter j. Assuming, without loss of generality, that at the center of each coil there is a magnetic sensor, the field measured by the sensor at the center of transmitter i transmitted by coil j can be expressed as:

$$meas_i^j = Rot[\varphi, \theta, \psi]_{sensor\ i}^T \, Rot[\varphi, \theta, \psi]_{transmitter\ j} \, bDipol[Rot[\varphi, \theta, \psi]_{tramsmitter\ j}^T (x_j - x_i, y_j - y_i, z_j - z_i)].\overline{m_J}$$

where $\overline{m}_J$ is the magnetic dipole vector for transmitter j.

This equation has twelve unknowns, six location variables and six orientation variables. A set of N transmitting coils will have a total of nine N unknowns (three location variables and six orientation variables, three for the transmitter and three for the sensor). Each sensor measuring the field from all the transmitters (excluding the one it is associated with) measures 3(N-1) values, for a total of 3 N (N-1) equations.

For any system with four or more coils the number of equations is greater than the number of unknowns. The system can also be solved using an optimization algorithm, with the cost function being the sum (sum of the squares) of the difference between the measurements and the right side of Equation 1. The condition that the origin of the coordinate system is the centroid of the coils centers is included as a constraint. If distances between coils are well known and fixed, they can be included as extra constraints.

It will be appreciated by persons skilled in the art that the present teachings are not limited to what has been particularly shown and described hereinabove. Rather, the scope of the present invention is defined by the appended claims and hence includes both combinations and sub-combinations of the various features described hereinabove, as well as variations and modifications thereof that are not in the prior art, which would occur to persons skilled in the art upon reading the foregoing description.

LIST OF ELEMENTS

| 24 | patient |
|---|---|
| 28 | support structure |
| 36 | catheter |
| 38 | heart |
| 40 | distal end of catheter |
| 42 | position sensor |
| 44 | console |
| 48 | tracking processor |
| 49 | connector cable |
| 50 | display |
| 100 | magnetic field location system |
| 201 | field generating coil unit |
| 202 | low-profile elongated field generation pad |
| 203 | clamping/securing mechanism |
| 204 | connecting rod |
| 205 | arm rest |
| 206 | securing mechanism arm |

207     adjustable coupling
208     table engaging arm portion
401     single axis coil
404     connecting rod members
501     clamp handle
502     pivot shaft
503     clamping end portion
801     elastic member
803     securing mechanism
804     boss
806     securing mechanism arm
808     table engaging arm portion
901     rack and pinion mechanism

**Claims**

1. A magnetic field location system : (100) for use in tracking a distal end (40) of a catheter (36) or the like, that includes a magnetic sensor (42), in a surgical or exploratory procedure within a living body disposed on a support structure, comprising:

   a first field generation pad (202) configured for selective placement along a first side of the living body disposed on the support structure (28) that includes a plurality of magnetic coils (201) operable to generate magnetic fields;
   a second field generation pad (202) configured for selective placement along an opposite side of the living body disposed on the support structure (28) that includes a plurality magnetic coils (201) operable to generate magnetic fields;
   a securing structure (203) configured to releasably secure the first and second field generation pads to the support structure (28) when selectively placed on the support structure (28) ;and
   a processor (48) coupled to the magnetic coils of the first and second field generation pads configured to execute a process for correlation of magnetic fields generated by the field generation pads (202) such that a reference magnetic field is defined that extends within a desired portion of the living body to enable precise location and tracking of a distal end (40) of a catheter (36) via the magnetic sensor (42) within the reference magnetic field.

2. The magnetic field location system of claim 1, wherein the first and second field generation pads are relatively symmetric and define relatively symmetric magnetic coil arrays when placed by the sides of the living body.

3. The magnetic field location system of claim 2 wherein the magnetic fields generated by the first and second field generation pads are generated by two magnetic coils disposed in each field generation pad at a defined distance from each other, where each magnetic coil is configured as a tri-axial coil unit.

4. The magnetic field location system of claim 3 wherein the tri-axial coil units are configured as tri-coil bobbins and have a combined weight of approximately 200 grams.

5. The magnetic field location system of claim 2 wherein the magnetic fields generated by the first and second field generation pads are generated by six linearly aligned and equally spaced magnetic coils disposed in each field generation pad, where each magnetic coil is configured as a single coil unit.

6. The magnetic field location system of claim 1 wherein each of the first and second field generation pads are configured with a height not exceeding 20 mm and a weight not exceeding 3 Kg and the securing structure is configured such that the first and second field generation pads are able to be selectively placed on and secured to the support structure in less than 60 seconds and are also enable to be removed from the support structure in less than 60 seconds..

7. The magnetic field location system of claim 1 wherein:

   the securing structure includes the first and second securing mechanisms attached to each field generation pad at predefined locations spaced apart from each other;
   each securing mechanism having an arm having:

an end attached to the respective field generation pad; and

a support structure engaging portion configured such that the support structure engaging portion engages a side of the support structure when the respective field generation pad is selectively placed on the support structure to thereby define the relative placement of the respective field generation pad on the support structure.

8. The magnetic field location system of claim 7 wherein each securing mechanism has a clamp configured to be operated to a clamping position from an open position to engage the respective support structure engaging portion with a side of the support structure when the respective field generation pad is selectively placed on the support structure.

9. The magnetic field location system of claim 7 wherein the end of the arm of each securing mechanism that is attached to the respective field generation pad has an adjustable coupling to enable the positioning of the respective support structure engaging portion to be positioned at an extended or retracted position relative to the respective field generation pad whereby the first and second field generation pads are placed furthest from each other and closest to the sides of the support structure when all of the support structure engaging portions are in the retracted position, and whereby the first and second field generation pads are placed closest to each other and furthest from the sides of the support structure when all of the support structure engaging portions are in the extended position.

10. The magnetic field location system of claim 7, wherein

the first and second securing mechanisms each have a coupling component configured for connection beneath the support structure; and

the securing structure includes a first joining member configured to attachment to the coupling components of the first securing mechanisms and a second joining member configured for attachment to the coupling components of the second securing mechanisms to secure the selective placement of the field generation pads on the support structure when the joining member are attached.

**Patentansprüche**

1. Magnetfeldlokalisierungssystem (100) zur Verwendung in der Verfolgung eines distalen Endes (40) eines Katheters (36) oder dergleichen, der einen Magnetsensor (42) umfasst, in einem chirurgischen oder explorativen Verfahren in einem lebenden Körper, der auf einer Tragstruktur angeordnet ist, umfassend:

ein erstes Felderzeugungspolster (202), das zur selektiven Platzierung entlang einer ersten Seite des auf der Tragstruktur (28) angeordneten lebenden Körpers ausgelegt ist, das eine Vielzahl von Magnetspulen (201) umfasst, die betreibbar sind, um Magnetfelder zu erzeugen;

ein zweites Felderzeugungspolster (202), das zur selektiven Platzierung entlang einer gegenüberliegenden Seite des auf der Tragstruktur (28) angeordneten lebenden Körpers ausgelegt ist, das eine Vielzahl von Magnetspulen (201) umfasst, die betreibbar sind, um Magnetfelder zu erzeugen;

eine Befestigungsstruktur (203), die dazu ausgelegt ist, das erste und das zweite Felderzeugungspolster beim selektiven Platzieren auf der Tragstruktur (28) an der Tragstruktur (28) zu befestigen; und

einen Prozessor (48), der mit den Magnetspulen des ersten und des zweiten Felderzeugungspolsters gekoppelt und dazu ausgelegt ist, einen Prozess zur Korrelation von Magnetfeldern auszuführen, die von den Felderzeugungspolstern (202) erzeugt wurden, sodass ein Referenzmagnetfeld definiert wird, das sich innerhalb eines gewünschten Abschnitts des lebenden Körpers erstreckt, um eine präzise Lokalisierung und Verfolgung eines distalen Endes (40) eines Katheters (36) über den Magnetsensor (42) innerhalb des Referenzmagnetfeldes zu ermöglichen.

2. Magnetfeldlokalisierungssystem nach Anspruch 1, wobei das erste und das zweite Felderzeugungspolster relativ symmetrisch sind und beim Platzieren an den Seiten des lebenden Körpers relativ symmetrische Magnetspulenarrays definieren.

3. Magnetfeldlokalisierungssystem nach Anspruch 2, wobei die von dem ersten und dem zweiten Felderzeugungspolster erzeugten Magnetfelder von zwei Magnetspulen erzeugt werden, die in jedem Felderzeugungspolster in einem definierten Abstand zueinander angeordnet sind, wobei jede Magnetspule als eine dreiachsige Spuleneinheit ausgelegt ist.

**4.** Magnetfeldlokalisierungssystem nach Anspruch 3, wobei die dreiachsigen Spuleneinheiten als Drei-Spulen-Bobbin ausgelegt sind und ein kombiniertes Gewicht von ungefähr 200 Gramm aufweisen.

**5.** Magnetfeldlokalisierungssystem nach Anspruch 2, wobei die von dem ersten und dem zweiten Felderzeugungspolster erzeugten Magnetfelder von sechs linear ausgerichteten und gleichmäßig beabstandeten Magnetspulen erzeugt werden, die in jedem Felderzeugungspolster angeordnet sind, wobei jede Magnetspule als eine Einzelspuleneinheit ausgelegt ist.

**6.** Magnetfeldlokalisierungssystem nach Anspruch 1, wobei jedes des ersten und des zweiten Felderzeugungspolsters mit einer Höhe ausgelegt ist, die 20 mm nicht übersteigt, und einem Gewicht, das 3 kg nicht übersteigt, und die Befestigungsstruktur derart ausgelegt ist, dass das erste und das zweite Felderzeugungspolster in weniger als 60 Sekunden selektiv auf der Tragstruktur platziert und daran befestigt werden und in weniger als 60 Sekunden von der Tragstruktur entfernt werden können.

**7.** Magnetfeldlokalisierungssystem nach Anspruch 1, wobei:

die Befestigungsstruktur den ersten und den zweiten Befestigungsmechanismus umfasst, die an jedem Felderzeugungspolster an vordefinierten voneinander beabstandeten Stellen angebracht sind;
wobei jeder Befestigungsmechanismus einen Arm aufweist, der aufweist:

ein Ende, das an dem jeweiligen Felderzeugungspolster angebracht ist; und
einen Tragstruktureingriffabschnitt, der derart ausgelegt ist, dass der Tragstruktureingriffabschnitt eine Seite der Tragstruktur in Eingriff nimmt, wenn das jeweilige Felderzeugungspolster selektiv auf der Tragstruktur platziert wird, um dadurch die relative Platzierung des jeweiligen Felderzeugungspolsters an der Tragstruktur zu definieren.

**8.** Magnetfeldlokalisierungssystem nach Anspruch 7, wobei jeder Befestigungsmechanismus eine Klemme aufweist, die dazu ausgelegt ist, aus einer offenen Position in eine Klemmposition betrieben zu werden, um den jeweiligen Tragstruktureingriffabschnitt mit einer Seite der Tragstruktur in Eingriff zu bringen, wenn das jeweilige Felderzeugungspolster selektiv auf der Tragstruktur platziert wird.

**9.** Magnetfeldlokalisierungssystem nach Anspruch 7, wobei das Ende des Arms eines jeden Befestigungsmechanismus, der an dem jeweiligen Felderzeugungspolster angebracht ist, eine einstellbare Kopplung aufweist, um zu ermöglichen, dass die Positionierung des jeweiligen Tragstruktureingriffabschnitts in einer ausgefahrenen oder eingefahrenen Position bezogen auf das jeweilige Felderzeugungspolster positioniert wird, wodurch das erste und das zweite Felderzeugungspolster voneinander am weitesten entfernt und den Seiten der Tragstruktur am nächsten gelegen platziert werden, wenn sich alle der Tragstruktureingriffabschnitte in der eingefahrenen Position befinden, und wodurch das erste und das zweite Felderzeugungspolster am nächsten zueinander und von den Seiten der Tragstruktur am weitesten entfernt platziert werden, wenn sich alle der Tragstruktureingriffabschnitte in der ausgefahrenen Position befinden.

**10.** Magnetfeldlokalisierungssystem nach Anspruch 7, wobei
der erste und der zweite Befestigungsmechanismus jeweils eine Kopplungskomponente aufweisen, die zur Verbindung unterhalb der Tragstruktur ausgelegt ist; und
die Befestigungsstruktur ein erstes Verbindungselement umfasst, das zur Befestigung an den Kopplungskomponenten des ersten Befestigungsmechanismus ausgelegt ist, und ein zweites Verbindungselement, das zur Befestigung an den Kopplungskomponenten des zweiten Befestigungsmechanismus ausgelegt ist, um beim Anbringen der Verbindungselemente die selektive Platzierung der Felderzeugungspolster auf der Tragstruktur zu befestigen.

**Revendications**

**1.** Système (100) de localisation par champs magnétiques destiné à être utilisé dans le suivi d'une extrémité distale (40) d'un cathéter (36) ou similaire, qui comprend un capteur magnétique (42), dans une procédure chirurgicale ou exploratoire à l'intérieur d'un corps vivant disposé sur une structure porteuse, comportant :

un premier coussin (202) de génération de champs configuré pour une mise en place sélective le long d'un premier côté du corps vivant disposé sur la structure porteuse (28) qui comprend une pluralité de bobines magnétiques (201) utilisables pour générer des champs magnétiques ;
un second coussin (202) de génération de champs configuré pour une mise en place sélective le long d'un côté opposé du corps vivant disposé sur la structure porteuse (28) qui comprend une pluralité de bobines magnétiques (201) utilisables pour générer des champs magnétiques ;
une structure (203) de fixation configurée pour fixer de façon libérable les premier et second coussins de génération de champs à la structure porteuse (28) lorsqu'ils sont placés sélectivement sur la structure porteuse (28) ; et
un processeur (48) couplé aux bobines magnétiques des premier et second coussins de génération de champs configuré pour exécuter un processus de corrélation de champs magnétiques générés par les coussins (202) de génération de champs de telle façon qu'un champ magnétique de référence soit défini, qui s'étend à l'intérieur d'une partie souhaitée du corps vivant pour permettre une localisation précise et un suivi d'une extrémité distale (40) d'un cathéter (36) via le capteur magnétique (42) à l'intérieur du champ magnétique de référence.

2. Système de localisation par champs magnétiques selon la revendication 1, les premier et second coussins de génération de champs étant relativement symétriques et définissant des batteries de bobines magnétiques relativement symétriques lorsqu'ils sont placés aux côtés du corps vivant.

3. Système de localisation par champs magnétiques selon la revendication 2 les champs magnétiques générés par les premier et second coussins de génération de champs étant générés par deux bobines magnétiques disposées dans chaque coussin de génération de champs à une distance définie l'une de l'autre, où chaque bobine magnétique est configurée comme une unité de bobine triaxiale.

4. Système de localisation par champs magnétiques selon la revendication 3, les unités de bobines triaxiales étant configurées comme des fuseaux à trois bobines et présentant un poids combiné d'environ 200 grammes.

5. Système de localisation par champs magnétiques selon la revendication 2, les champs magnétiques générés par les premier et second coussins de génération de champs étant générés par six bobines magnétiques linéairement alignées et uniformément espacées disposées dans chaque coussin de génération de champs, où chaque bobine magnétique est configurée comme une unité de bobine unique.

6. Système de localisation par champs magnétiques selon la revendication 1, chacun des premier et second coussins de génération de champs étant configuré avec une hauteur ne dépassant pas 20 mm et un poids ne dépassant pas 3 kg et la structure de fixation étant configurée de telle façon que les premier et second coussins de génération de champs puissent être sélectivement placés sur et fixés à la structure porteuse en moins de 60 secondes et puissent également être retirés de la structure porteuse en moins de 60 secondes.

7. Système de localisation par champs magnétiques selon la revendication 1 :

la structure de fixation comprenant les premiers et seconds mécanismes de fixation fixés à chaque coussin de génération de champs en des emplacements prédéfinis espacés les uns par rapport aux autres ;
chaque mécanisme de fixation étant doté d'un bras présentant :

une extrémité fixée au coussin respectif de génération de champs ; et
une partie d'interaction avec la structure porteuse configurée de telle façon que la partie d'interaction avec la structure porteuse interagisse avec un côté de la structure porteuse lorsque le coussin respectif de génération de champs est placé sélectivement sur la structure porteuse pour définir ainsi le positionnement relatif du coussin respectif de génération de champs sur la structure porteuse.

8. Système de localisation par champs magnétiques selon la revendication 7, chaque mécanisme de fixation étant doté d'une pince configurée pour être actionnée jusqu'à une position de serrage en partant d'une position ouverte pour faire interagir la partie respective d'interaction avec la structure porteuse avec un côté de la structure porteuse lorsque le coussin respectif de génération de champs est placé sélectivement sur la structure porteuse.

9. Système de localisation par champs magnétiques selon la revendication 7, l'extrémité du bras de chaque mécanisme de fixation qui est fixée au coussin respectif de génération de champs étant dotée d'un accouplement réglable pour permettre le positionnement de la partie respective d'interaction avec la structure porteuse dans une position déployée ou rétractée par rapport au coussin respectif de génération de champs, les premier et second coussins de génération de champs étant ainsi placés le plus loin l'un de l'autre et le plus près des côtés de la structure porteuse lorsque toutes les parties d'interaction avec la structure porteuse sont dans la position rétractée, et les premier et second coussins de génération de champs étant ainsi placés le plus près l'un de l'autre et le plus loin des côtés de la structure porteuse lorsque toutes les parties d'interaction avec la structure porteuse sont dans la position déployée.

10. Système de localisation par champs magnétiques selon la revendication 7, les premiers et seconds mécanismes de fixation étant dotés chacun d'un composant d'accouplement configuré en vue d'une liaison sous la structure porteuse ; et
la structure de fixation comprenant un premier élément de jonction configuré pour se fixer aux composants d'accouplement des premiers mécanismes de fixation et un second élément de jonction configuré pour se fixer aux composants d'accouplement des seconds mécanismes de fixation afin d'assurer la mise en place sélective des coussins de génération de champs sur la structure porteuse lorsque les éléments de jonction sont fixés.

FIG. 1

FIG. 2A

FIG. 2B

FIG. 2C

FIG. 3

FIG. 4A

FIG. 4B

**FIG. 5A**

**FIG. 5B**

202

206

208

203

28

## FIG. 6A

206

202

208

503

502

203

501

28

## FIG. 6B

FIG. 7

FIG. 8A

FIG. 8B

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6618612 B
- US 5391199 A
- US 6690963 B
- US 6484118 B
- US 6239724 B
- US 6332089 B
- WO 9605768 A
- US 20020065455 A1
- US 20030120150 A1
- US 20040068178 A1
- EP 0883374 B1
- US 20170196477 A1
- US 20060025668 A1
- EP 1570781 A1